# FASCICULE DE BREVET EUROPEEN

(11) **EP 0 713 696 B1**
(45) Date de publication et mention de la délivrance du brevet: **21.03.2001**
(21) Numéro de dépôt: 95402182.0
(22) Date de dépôt: 28.09.1995
(51) Int. Cl.: A61K 7/48, A61K 7/06, A61K 47/46

(54) **Produit pour application topique contenant une lipase et un precurseur d'hydroxyacide**
Mittel zur topischen Verwendung enthaltend eine Lipase und einen Hydroxysäurevorlaufer
Topical application product containing a lipase and a precursor of hydroxyacid

(30) Priorité: 24.10.1994 FR 9412685
(43) Date de publication de la demande: 29.05.1996
(73) Titulaire: L'OREAL, 75008 Paris (FR)
(72) Inventeur: Maurin, Emmanuelle, F-95290 L'Isle-Adam (FR); Sera, Daniel, F-94150 L'Hay-les-Roses (FR); Guth, Gérard, F-95160 Montmorency (FR)
(74) Mandataire: Lhoste, Catherine

(56) Documents cités:
- EP-A- 0 599 819
- WO-A-83/03061
- FR-A- 2 211 209
- FR-A- 2 556 218
- PATENT ABSTRACTS OF JAPAN vol. 12 no. 333 (C-526) [3180] & JP-A-63 096109 (SHISEIDO)
- PATENT ABSTRACTS OF JAPAN vol. 14 no. 294 (C-732) [4237] & JP-A-02 096510 (KANEBO)

## Description

La présente invention a pour objet un produit pour application topique apte à libérer sur la peau un hydroxyacide, et son utilisation pour traiter en douceur la peau, y compris le cuir chevelu. Elle se rapporte aussi à l'utilisation de ce produit et à un procédé pour le traitement cosmétique et/ou dermatologique de la peau, et notamment pour le traitement de l'acné, des rides et des ridules.

Les hydroxyacides sont de plus en plus utilisés dans les domaines cosmétique ou dermatologique pour le soin du visage et/ou du corps et plus spécialement pour donner au visage un teint lumineux et éclatant et donc une bonne mine, un aspect lisse et plus jeune, ainsi que pour faire disparaître les comédons dûs à l'acné (G. A. NOWAK, "Galenische Versuche zur Formulierung von α-Hydroxysaüren enthaltenden kosmetischen Mitteln", Parfümerie und Kosmetik, 74. Jahrgang, Nr. 9/93).

Malheureusement, ces composés acides sont généralement proposés dans des compositions ayant un pH inférieur ou égal à 4 en vue de maintenir actifs ces acides, et l'application de ces compositions sur la peau entraîne l'inconvénient majeur de provoquer des picotements, des démangeaisons, des tiraillements pouvant conduire à un important inconfort. L'utilisation de ces compositions acides pour les utilisateurs à peau sensible est donc souvent rédhibitoire.

Aussi, il subsiste le besoin d'un produit cosmétique et/ou dermatologique à base d'hydroxyacides conférant notamment à la peau cette bonne mine et ce rajeunissement, et capable de traiter l'acné, sans entraîner ces inconvénients.

La demanderesse a maintenant trouvé de manière inattendue que l'utilisation d'un enzyme particulier, la lipase, associé à des esters d'hydroxyacides, permettait d'avoir une libération progressive de l'hydroxyacide sur la peau, de supprimer ainsi le caractère agressif des hydroxyacides et d'éviter par conséquent les désavantages de l'art antérieur.

Aussi, la présente invention a pour objet un produit pour application topique contenant un enzyme et au moins un précurseur d'un hydroxyacide, caractérisé en ce que l'enzyme est une lipase et en ce que le précurseur est un ester comportant au moins une fonction ester à chaîne linéaire ou ramifiée saturée ou insaturée, ayant de 2 à 25 atomes de carbone.

Par produit, on entend aussi bien la partie cosmétique ou dermatologique (ensemble de la composition) que le dispositif commercial comportant un récipient dans lequel sont logés l'enzyme et le précurseur isolés l'un de l'autre.

L'utilisation de l'association ester d'hydroxyacide / lipase permet non seulement une libération ménagée de l'hydroxyacide, et donc une agressivité moindre, mais aussi une facilité de formulation du fait d'une meilleure compatibilité des esters avec les milieux couramment utilisés dans les domaines cosmétique ou dermatologique. En particulier, le produit de l'invention peut être utilisé sans inconvénient par les personnes à peau sensible, notamment du fait de la possibilité de formuler à un pH voisin de 6.

La lipase est un enzyme connu pour hydrolyser les triglycérides en mono- et diglycérides, en glycérol et en acides gras libres. Elle est en particulier utilisée dans les détergents (voir l'article "Lipases as detergent components", H. Andree et al., Journal of Applied Biochemistry, 1980, vol. 2, pages 218 à 229), afin de permettre l'élimination des taches graisseuses telles que celles provenant des matières grasses de friture, d'huiles, de sebum ou de cosmétiques gras comme les rouges à lèvres. Du fait de sa propriété de couper les triglycérides, elle a été utilisée en cosmétique sous forme immobilisée pour nettoyer la peau (voir par exemple US-A-4556554).

La lipase selon l'invention doit être suffisamment stable pour conserver son activité enzymatique. Elle appartient au groupe des enzymes de classification EC 3.1.1.3., ce qui correspond à une lipase qui coupe les liaisons ester en position 1 et 3 d'un triglycéride. Elle peut être choisie par exemple parmi celles vendues sous les dénominations commerciales "lipase SP644" et "lipolase 100 L" par la société Novo Nordisk.

La lipase est utilisée dans le produit selon l'invention en une quantité allant de 0,05 à 30 % en poids, de préférence de 0,1 à 10 % en poids, et mieux de 0,1 à 5 % en poids, par rapport au poids total de la composition.

Les hydroxyacides auxquels s'applique l'invention peuvent être des α-hydroxyacides ou des β-hydroxyacides, qui peuvent être linéaires, ramifiés ou cycliques, saturés ou insaturés. Les atomes d'hydrogène de la chaîne carbonée peuvent, en outre, être substitués par des halogènes, des radicaux halogénés, alkylés, acylés, acyloxylés, alcoxy carbonylés ou alcoxylés ayant de 2 à 18 atomes de carbone.

Les hydroxyacides les plus utilisés en cosmétique sont notamment les acides glycolique, lactique, malique, tartrique, citrique, hydroxy-2 alcanoïque, mandélique, salicylique, ainsi que leurs dérivés alkylés en C₁ à C₂₂ comme l'acide n-octanoyl-5-salicylique, l'acide n-dodécanoyl-5-salicylique, l'acide 2-hydroxy-3-méthyl-benzoïque, ou encore leurs dérivés alcoxylés en C₁ à C₂₂ comme l'acide 2-hydroxy-3-méthoxybenzoïque.

L'ester utilisé selon l'invention est un ester comportant une ou plusieurs fonctions ester à chaîne linéaire ou ramifiée, saturée ou insaturée, ayant de 2 à 25 atomes de carbone, comportant éventuellement un ou plusieurs substituants. La chaîne de la fonction ester est choisie en particulier parmi les radicaux acyle, benzoyle, alkylbenzoyle, acylbenzoyle, 2-hydroxyphénylacétyle, éventuellement substitués. Le substituant peut être en particulier un radical hydroxylé.

Selon une réalisation préférée de l'invention, la chaîne de la fonction ester a de 12 à 18 atomes de carbone.

Il s'agit par exemple d'un ester choisi parmi les esters d'alcools gras tels que les alcools dodécylique, hexadécylique, stéarylique, cétylique, myristylique, linoléylique, octylique, oléique, ou également d'esters d'alcools butylique, propylique, éthylique, ou encore les esters de polyols tels que les esters de propylène glycol, de butylène glycol, d'éthylène glycol ou de glycérol, ou des mélanges de ces esters.

Selon une réalisation préférée de l'invention, l'ester utilisé est le salicylate de cétyle, le salicylate de dodécyle, le lactate de cétyle, le glycolate de propyle, le lactate de glycéryle, le salicylate de menthyle, le lactate de menthyle.

L'ester est utilisé dans le produit selon l'invention en une quantité allant de 0,1 à 20 % en poids, et de préférence de 1 à 10 % en poids par rapport au poids total de la composition.

Selon une première variante de l'invention, on introduit la lipase et le précurseur dans une composition unique, qui est, de préférence, préparée juste avant l'emploi.

Selon une seconde variante, la lipase et le précurseur sont conditionnés de sorte à ne pas être en contact l'un avec l'autre, par exemple dans deux compositions différentes, qui peuvent être soit mélangées au moment de l'application soit appliquées de façon successive ou décalée dans le temps.

On peut par exemple disposer les compositions dans deux compartiments qui sont en communication avec un conduit commun, d'où elles peuvent sortir en se mélangeant avant application sur la peau. De tels dispositifs de conditionnement en deux compartiments sont par exemple décrits dans les documents FR-A-2045559, FR-A-2105332, FR-A-2258319, FR-A-2293375, FR-A-2586913 ou FR-A-2643615.

On peut aussi réaliser l'une des compositions sous forme encapsulée et/ou sous forme de microcapsules ou de microgranulés immergés dans l'autre composition, les microcapsules ou les microgranulés étant écrasés au moment de l'application par frottement sur la peau, ce qui permet ainsi le mélange de la lipase et du précurseur et la libération de l'actif libre sur la peau.

Le produit selon l'invention peut être utilisé, selon l'ester d'actif qu'il contient, pour le traitement cosmétique et/ou dermatologique de la peau.

Le produit selon l'invention comporte avantageusement un milieu approprié pour une application topique, destiné aux domaines cosmétique et/ou dermatologique.

Aussi, l'invention a en outre pour objet l'utilisation du produit tel que défini ci-dessus pour préparer une pommade ou un onguent dermatologique destiné au traitement thérapeutique de la peau.

L'invention a encore pour objet l'utilisation du produit tel que défini ci-dessus pour le traitement cosmétique de la peau. En particulier, l'invention a pour objet l'utilisation du produit tel que défini ci-dessus pour lutter contre l'acné, les rides et les ridules, de façon non thérapeutique.

L'invention a encore pour objet un procédé de traitement cosmétique de la peau consistant à appliquer sur la peau de façon simultanée ou décalée un enzyme et au moins un précurseur d'un hydroxyacide, caractérisé en ce que que l'enzyme est une lipase et en ce que le précurseur est un ester comportant au moins une fonction ester à chaîne linéaire ou ramifiée, saturée ou insaturée, ayant de 2 à 25 atomes de carbone.

Le milieu cosmétiquement et/ou dermatologiquement acceptable comprend généralement de l'eau, ou un mélange d'eau et de corps gras, ou un mélange de corps gras.

Comme corps gras utilisables dans l'invention, on peut citer les huiles minérales (vaseline, huile minérale), les huiles végétales et leurs dérivés hydrogénés, les huiles animales, les huiles de synthèse, les huiles siliconées (diméthicone, cyclométhicone) et les huiles fluorées. Comme autres corps gras, on peut encore citer les alcools gras (alcool cétylique, alcool stéarylique), les acides gras et les cires.

En particulier, le produit peut se présenter sous forme de solutions aqueuses, alcooliques ou hydroalcooliques, de gels hydrophiles ou lipophiles, de microémulsions, d'émulsions eau-dans-huile ou huile-dans-eau ou eau-dans-huile-dans-eau ou huile-dans-eau-dans-huile ayant l'aspect d'une crème ou d'un gel, éventuellement aptes à mousser, sous forme d'aérosol, ou encore sous forme de dispersions vésiculaires contenant des lipides ioniques et/ou non ioniques. Ces formes galéniques sont préparées selon les méthodes usuelles des domaines considérés.

De façon connue, le milieu approprié pour une application topique selon l'invention peut contenir également des adjuvants habituels dans le domaine cosmétique ou dermatologique, tels que les gélifiants hydrophiles ou lipophiles, les tensioactifs, les actifs hydrophiles ou lipophiles, les conservateurs, les antioxydants, les solvants, les parfums, les charges, les filtres et les matières colorantes.

Les quantités des différents constituants du produit selon l'invention sont celles classiquement utilisées dans les domaines considérés.

Le produit selon l'invention peut constituer notamment des compositions de protection, de traitement ou de soin pour le visage, pour le cou, pour les mains ou pour le corps, des compositions pour les cheveux, et notamment pour le soin du cuir chevelu, par exemple sous forme de shampooings, de lotions traitantes, de crèmes ou de gels coiffants, de lotions ou de gels antichute.

Les exemples qui suivent sont donnés à titre illustratif afin de mieux faire comprendre l'invention. Les quantités indiquées sont des pourcentages en poids.

### Exemple 1 : Crème de soin

| A. Emulsion contenant l'ester d'hydroxyacide : | |
|---|---|
| *Phase huileuse :* | |
| Steareth-2 (tensioactif) | 3 % |
| Steareth-21 (tensioactif) | 2 % |
| PPG-15 stearyl éther (tensioactif) | 9 % |
| Alcool cétylique | 2 % |
| Alcool stéarylique | 1,5 % |
| Vaseline | 5 % |
| Salicylate de dodécyle | 2 % |
| Cyclométhicone | 3 % |

| *Phase aqueuse :* | |
|---|---|
| Propylène glycol (hydratant) | 4 % |
| PEG-20 | 5 % |
| Phénoxyéthanol (conservateur) | 0,5 % |
| Eau | qsp 100 % |

| B. Emulsion contenant la lipase : | |
|---|---|
| *Phase huileuse :* | |
| Steareth-2 (tensioactif) | 3 % |
| Steareth-21 (tensioactif) | 2 % |
| PPG-15 stearyl éther (tensioactif) | 9 % |
| Alcool cétylique | 2 % |
| Alcool stéarylique | 1,5 % |
| Vaseline | 5 % |
| Cyclométhicone | 3 % |

| *Phase aqueuse :* | |
|---|---|
| Propylène glycol (hydratant) | 4 % |
| PEG-20 | 5 % |
| Phénoxyéthanol (conservateur) | 0,5 % |
| Lipolase 100 L | 8 % |
| Eau | qsp 100 % |

Les émulsions A et B sont disposées dans deux compartiments séparés et mélangées au moment de l'application sur la peau.

### Exemple 2 : Crème de soin

| A. Emulsion contenant l'ester d'hydroxyacide : | |
|---|---|
| *Phase huileuse :* | |
| Triceteareth-4 phosphate/sodium C₁₄₋₁₇ Alkyl sec sulfonate (Hostacerin CG de Hoechst Celanese) (tensioactif) | 10 % |
| Alcool cétylique | 1 % |
| Alcool stéarylique | 1,5 % |
| Vaseline | 2 % |
| Huile minérale | 4 % |
| Diméthicone (Silicone L45 de Union Carbide) | 3 % |
| Salicylate de dodécyle | 2 % |
| Cyclométhicone | 3 % |

| *Phase aqueuse:* | |
|---|---|
| Propylène glycol(hydratant) | 2 % |
| PEG-20 | 2 % |
| Phénoxyéthanol (conservateur) | 0,4 % |
| Eau | qsp 100 % |

| B. Emulsion contenant la lipase : | |
|---|---|
| *Phase huileuse :* | |
| Triceteareth-4 phosphate/sodium C₁₄₋₁₇ Alkyl sec sulfonate (Hostacerin CG de Hoechst Celanese) (tensioactif) | 10 % |
| Alcool stéarylique | 1 % |
| Vaseline | 2 % |
| Huile minérale | 4 % |
| Diméthicone (Silicone L45 de Union Carbide) | 3 % |
| Cyclométhicone | 3 % |

| *Phase aqueuse :* | |
|---|---|
| Propylène glycol (hydratant) | 2 % |
| PEG-20 | 2 % |
| Phénoxyéthanol (conservateur) | 0,4 % |
| Lipolase SP 644 | 8 % |
| Eau | qsp 100 % |

Les émulsions A et B sont disposées dans deux compartiments séparés et mélangées au moment de l'application sur la peau.

## Revendications

1. Produit pour application topique contenant un enzyme et au moins un précurseur d'un hydroxyacide, caractérisé en ce que l'enzyme est une lipase et en ce que le précurseur est un ester comportant au moins une fonction ester à chaîne linéaire ou ramifiée saturée ou insaturée, ayant de 2 à 25 atomes de carbone.

2. Produit cosmétique selon la revendication 1, caractérisé en ce que la chaîne de la fonction ester est choisie parmi les radicaux acyle, benzoyle, alkylbenzoyle, acylbenzoyle, 2-hydroxyphénylacétyle, éventuellement substitués.

3. Produit selon la revendication 1 ou 2, caractérisé en ce que la chaîne de la fonction ester a de 12 à 18 atomes de carbone.

4. Produit selon l'une quelconque des revendications précédentes, caractérisé en ce que l'ester est choisi parmi les esters d'alcool dodécylique, d'alcool hexadécylique, d'alcool stéarylique, d'alcool cétylique, d'alcool myristylique, d'alcool linoléylique, d'alcool octylique, d'alcool oléique, d'alcool butylique, d'alcool propylique, d'alcool éthylique, de polyol, ou leurs mélanges.

5. Produit selon l'une quelconque des revendications précédentes, caractérisé en ce que l'hydroxyacide est choisi dans le groupe comprenant les α-hydroxyacides ou les β-hydroxyacides.

6. Produit l'une quelconque des revendications précédentes, caractérisé en ce que l'hydroxyacide est choisi dans le groupe comprenant les acides glycolique, lactique, malique, tartrique, citrique, hydroxy-2 alcanoïque, mandélique, salicylique, ainsi que leurs dérivés alkylés en C₁ à C₂₂ ou leurs dérivés alcoxylés en C₁ à C₂₂.

7. Produit selon l'une quelconque des revendications précédentes, caractérisé en ce que l'enzyme et le précurseur sont conditionnés de sorte à ne pas être en contact l'un avec l'autre.

8. Produit selon la revendication 7, caractérisé en ce que l'enzyme et le précurseur sont conditionnés dans des compartiments séparés.

9. Produit selon l'une quelconque des revendications précédentes, caractérisé en ce que l'enzyme et/ou le précurseur sont sous forme encapsulée.

10. Produit selon l'une quelconque des revendications précédentes, caractérisé en ce que l'enzyme et/ou le précurseur sont sous forme de microcapsules ou de microgranulés.

11. Produit selon l'une quelconque des revendications précédentes, caractérisé en ce que la lipase est choisie dans le groupe des enzymes de classification EC 3.1.1.3.

12. Produit selon l'une quelconque des revendications précédentes, caractérisé en ce que l'enzyme est présent en une quantité allant de 0,05 à 30 % en poids par rapport au poids total du produit.

13. Produit selon l'une quelconque des revendications précédentes, caractérisé en ce que l'enzyme est présent en une quantité allant de 0,1 à 10 % en poids par rapport au poids total du produit.

14. Produit selon l'une quelconque des revendications précédentes, caractérisé en ce que le précurseur est présent en une quantité allant de 0,1 à 20 % en poids par rapport au poids total du produit.

15. Utilisation du produit selon l'une quelconque des revendications précédentes pour préparer une pommade ou un onguent dermatologique destiné au traitement thérapeutique de la peau.

16. Utilisation du produit selon l'une quelconque des revendications 1 à 14 pour le traitement cosmétique de la peau.

17. Utilisation du produit selon l'une quelconque des revendications 1 à 14 pour lutter contre l'acné, les rides et les ridules, de façon non thérapeutique.

18. Procédé de traitement cosmétique de la peau consistant à appliquer sur la peau de façon simultanée ou décalée un enzyme et au moins un précurseur d'un hydroxyacide, caractérisé en ce que que l'enzyme est une lipase et en ce que le précurseur est un ester comportant au moins une fonction ester à chaîne linéaire ou ramifiée, saturée ou insaturée, ayant de 2 à 25 atomes de carbone.

## Patentansprüche

1. Produkt zur topischen Anwendung, das mindestens ein Enzym und mindestens einen Precursor einer Hydroxysäure enthält, dadurch gekennzeichnet, daß das Enzym eine Lipase und der Precursor ein Ester ist, der mindestens eine Estergruppe mit linearer oder verzeigter, gesättigter oder ungesättigter Kette mit 2 bis 25 Kohlenstoffatomen aufweist.

2. Kosmetisches Produkt nach Anspruch 1, dadurch gekennzeichnet, daß die Kette der Estergruppe unter den Gruppen Acyl, Benzoyl, Alkylbenzoyl, Acylbenzoyl und 2-Hydroxyphenylacetyl, die gegebenenfalls substituiert sind, ausgewählt ist.

3. Produkt nach Anspruch 1 oder 2, dadurch gekennzeichnet, daß die Kette der Estergruppe 12 bis 18 Kohlenstoffatome aufweist.

4. Produkt nach einem der vorhergehenden Ansprüche, dadurch gekennzeichnet, daß der Ester unter den Estern von Dodecylalkohol, Hexadecylalkohol, Stearylalkohol, Cetylalkohol, Myristylalkohol, Linoleylalkohol, Octylalkohol, Butylalkohol, Propylalkohol, Ethylalkohol oder Polyolen oder deren Gemischen ausgewählt ist.

5. Produkt nach einem der vorhergehenden Ansprüche, dadurch gekennzeichnet, daß die Hydroxysäure unter den α-Hydroxysäuren oder den β-Hydroxysäuren ausgewählt ist.

6. Produkt nach einem der vorhergehenden Ansprüche, dadurch gekennzeichnet, daß die Hydroxysäure unter Glykolsäure, Milchsäure, Äpfelsäure, Weinsäure, Citronensäure, 2-Hydroxyalkansäuren, Mandelsäure, Salicylsäure sowie deren C₁₋₂₂-Alkylderivaten oder C₁₋₂₂-Alkoxyderivaten ausgewählt ist.

7. Produkt nach einem der vorhergehenden Ansprüche, dadurch gekennzeichnet, daß das Enzym und der Precursor so konfektioniert sind, daß sie nicht miteinander in Kontakt stehen.

8. Produkt nach Anspruch 7, dadurch gekennzeichnet, daß das Enzym und der Precursor in getrennten Abteilungen konfektioniert sind.

9. Produkt nach einem der vorhergehenden Ansprüche, dadurch gekennzeichnet, daß das Enzym und/oder der Precursor eingekapselt sind.

10. Produkt nach einem der vorhergehenden Ansprüche, dadurch gekennzeichnet, daß das Enzym und/oder der Precursor in Form von Mikrokapseln oder Mikrogranulat vorliegen.

11. Produkt nach einem der vorhergehenden Ansprüche, dadurch gekennzeichnet, daß die Lipase unter den Enzymen der Klassifizierung EC 3.1.1.3 ausgewählt ist.

12. Produkt nach einem der vorhergehenden Ansprüche, dadurch gekennzeichnet, daß das Enzym in einer Menge von 0,05 bis 30 Gew.-%, bezogen auf das Gesamtgewicht des Produktes, vorliegt.

13. Produkt nach einem der vorhergehenden Ansprüche, dadurch gekennzeichnet, daß das Enzym in einer Menge von 0,1 bis 10 Gew.-%, bezogen auf das Gesamtgewicht des Produktes, vorliegt.

14. Produkt nach einem der vorhergehenden Ansprüche, dadurch gekennzeichnet, daß der Precursor in einer Menge von 0,1 bis 20 Gew.-%, bezogen auf das Gesamtgewicht des Produktes, vorliegt.

15. Verwendung eines Produktes nach einem der vorhergehenden Ansprüche zur Herstellung einer Salbe oder dermatologischen Salbe zur therapeutischen Behandlung der Haut.

16. Verwendung eines Produktes nach einem der Ansprüche 1 bis 14 zur kosmetischen Behandlung der Haut.

17. Verwendung eines Produktes nach einem der Ansprüche 1 bis 14 zur nicht therapeutischen Bekämpfung von Akne, Falten und Fältchen.

18. Verfahren zur kosmetischen Behandlung der Haut, das darin besteht, auf die Haut gleichzeitig oder nacheinander ein Enzym und mindestens einen Precursor einer Hydroxysäure aufzubringen, dadurch gekennzeichnet, daß das Enzym eine Lipase und der Precursor ein Ester ist, der mindestens eine Estergruppe mit linearer oder verzeigter, gesättigter oder ungesättigter Kette mit 2 bis 25 Kohlenstoffatomen aufweist.

## Claims

1. Product for topical application containing an enzyme and at least one precursor of a hydroxy acid, characterized in that the enzyme is a lipase and in that the precursor is an ester containing at least one ester functional group with a saturated or unsaturated, linear or branched chain having from 2 to 25 carbon atoms.

2. Cosmetic product according to Claim 1, characterized in that the chain of the ester functional group is chosen from acyl, benzoyl, alkylbenzoyl, acylbenzoyl and 2-hydroxyphenylacetyl radicals, which are optionally substituted.

3. Product according to Claim 1 or 2, characterized in that the chain of the ester functional group has from 12 to 18 carbon atoms.

4. Product according to any one of the preceding claims, characterized in that the ester is chosen from esters of dodecyl alcohol, hexadecyl alcohol, stearyl alcohol, cetyl alcohol, myristyl alcohol, linoleyl alcohol, octyl alcohol, oleyl alcohol, butyl alcohol, propyl alcohol, ethyl alcohol, polyol or their mixtures.

5. Product according to any one of the preceding claims, characterized in that the hydroxy acid is chosen from the group comprising α-hydroxy acids or β-hydroxy acids.

6. Product according to any one of the preceding claims, characterized in that the hydroxy acid is chosen from the group comprising glycolic, lactic, malic, tartaric, citric, 2-hydroxyalkanoic, mandelic and salicylic acids and their C₁ to C₂₂ alkylated derivatives or their C₁ to C₂₂ alkoxylated derivatives.

7. Product according to any one of the preceding claims, characterized in that the enzyme and the precursor are packaged so as not to be in contact with one another.

8. Product according to Claim 7, characterized in that the enzyme and the precursor are packaged in separate compartments.

9. Product according to any one of the preceding claims, characterized in that the enzyme and/or the precursor are in an encapsulated form.

10. Product according to any one of the preceding claims, characterized in that the enzyme and/or the precursor are in the form of microcapsules or of microgranules.

11. Product according to any one of the preceding claims, characterized in that the lipase is chosen from the group of enzymes of EC 3.1.1.3. classification.

12. Product according to any one of the preceding claims, characterized in that the enzyme is present in an amount ranging from 0.05 to 30 % by weight with respect to the total weight of the product.

13. Product according to any one of the preceding claims, characterized in that the enzyme is present in an amount ranging from 0.1 to 10 % by weight with respect to the total weight of the product.

14. Product according to any one of the preceding claims, characterized in that the precursor is present in an amount ranging from 0.1 to 20 % by weight with respect to the total weight of the product.

15. Use of the product according to any one of the preceding claims for preparing a dermatological salve or ointment intended for the therapeutic treatment of the skin.

16. Use of the product according to any one of Claims 1 to 14 for the cosmetic treatment of the skin.

17. Use of the product according to any one of Claims 1 to 14 for combating acne, wrinkles and fine lines non-therapeutically.

18. Process for the cosmetic treatment of the skin which consists in applying to the skin, simultaneously or with a time delay, an enzyme and at least one precursor of a hydroxy acid, characterized in that the enzyme is a lipase and in that the precursor is an ester containing at least one ester functional group with a saturated or unsaturated, linear or branched chain having from 2 to 25 carbon atoms.
